# EUROPÄISCHE PATENTSCHRIFT

(11) **EP 1 957 037 B1**
(45) Veröffentlichungstag und Bekanntmachung des Hinweises auf die Patenterteilung: **21.04.2010**
(21) Anmeldenummer: 01988082.2
(22) Anmeldetag: 27.12.2001
(51) Int. Cl.: A61K 9/00, A61K 47/36, A61K 31/4468

(54) **PHARMAZEUTISCHE ZUSAMMENSETZUNG ZUR NASALEN ANWENDUNG VON FENTANYL**
PHARMACEUTICAL COMPOSITION FOR NASAL APPLICATION OF FENTANYL
COMPOSITION PHARMACEUTIQUE DE FENTANYL POUR ADMINISTRATION NASALE

(30) Priorität: 22.12.2000 DE 10064219
(43) Veröffentlichungstag der Anmeldung: 20.08.2008
(73) Patentinhaber: Nasalis Pain Relief International GmbH, 58840 Plettenberg (DE)
(72) Erfinder: KLÖCKER, Norbert, 65232 Taunusstein (DE)
(74) Vertreter: Kailuweit, Frank
(86) Internationale Anmeldenummer: PCT/EP2001/015344
(87) Internationale Veröffentlichungsnummer: WO 2002/051380

(56) Entgegenhaltungen:
- WO-A-91/11992
- WO-A-94/10987
- WO-A-99/15150
- WO-A-99/16419
- WO-A-99/40932
- WO-A-99/62546
- JP-A- 10 279 421
- DATABASE WPI Week 199901 Derwent Publications Ltd., London, GB; AN 1999-005109 XP002199812 & JP 10 279421 A (SHISEIDO), 20. Oktober 1998 (1998-10-20)
- Summary report On the nasal tolerability assesed by in-vitro determination of cytotoxicity and of ex vivo in vitro ciliar activity of a new Fentanyl Nasal Spray.

## Beschreibung

Die Erfindung betrifft eine pharmazeutische Zusammensetzung zur nasalen Anwendung Applikation bestehend aus Fentanyl als Wirkstoff, Hyaluronsäure und/oder ihren Salzen- und gegebenenfalls mindestens einem Lösungsvermittler und/oder Hilfsstoff, wobei die Zytotoxizität der pharmakologisch wirksamen Substanz herabgesetzt, bzw. aufgehoben wird.

Insbesondere betrifft die Erfindung pharmazeutische Zusammensetzungen zur nasalen Applikation. Die auf dem Markt erhältlichen Arzneimittel zur nasalen Anwendung, seien es Pump- und Ventilsprays, sowie Nasentropfen beinhalten zumeist Wirkstoffe in wässriger Lösung. Das Problem dieser wässrigen Lösungen ist, dass fast alle gebräuchlichen und zugelassenen pharmakologisch wirksamen Substanzen, so auch Fentanyl, zytotoxisch sind und die Ziliarfunktion und somit die Clearancefunktion der Nasenschleimhaut beeinträchtigen. Wässrige Lösungen sind zudem hinsichtlich ihrer Stabilität relativ problematisch.

WO 94/10 987 offenbart eine Nasensprayformulierung die Fentanyl Citrat, Natrium glykocholat und wasser enthält.

Die starke pH-Wert-Abhängigkeit der nasalen Resorption von Wirkstoffen aus wässrigen Lösungen stellt ein weiteres Problem dar. Das optimale Milieu für die Zilien der Nasenschleimhaut liegt bei einem pH-Wert zwischen 7 und 9. Allerdings erfolgt eine maximale Resorption bei einem pH-Wert < 6. Ein pH-Wert von 4 führt zur Lähmung bis zur Zerstörung der Zilien. Zudem führt das brennende Gefühl in der Nase zu einer Minderung der Compliance und somit zu einem geringeren Therapieerfolg.

Aus dem Stand der Technik sind verschiedene Verwendungen von Hyaluronsäure in pharmazeutischen Zusammensetzungen bekannt. WO 91/11992 A offenbart die Verwendung von Hyaluronsäure oder deren Salzen in einer fließfähigen Lösung als Träger für das Einbringen von Medikamenten, typischerweise Antibiotika, in die Bauchhöhle WO 99/62546 A lehrt eine Impfstoff-Zusammensetzung ("Vaccine") aus Hyaluronsäureestern und einem Antigen zur mucosalen Verabreichung. WO 99/40932 offenbart eine mizellare pharmazeutische Zusammensetzung, die folgende Inhaltsstoffe umfasst: ein Wirkstoff in mizellarer Form; Wasser; ein Alkalimetallalkylsulfat, EDTA, mindestens ein Alkalimetallsalicylat und mindestens eine Mizellen bildende, absorptionsverstärkende Substanz. In WO 99/15150 A ist ein Freisetzungssystem offenbart, das zur verzögerten Wirkstofffreisetzung von pharmazeutischen Zusammensetzungen genutzt werden kann. Das Freisetzungssystem besteht insbesondere aus einer anorganischen Komponente (Calciumsulfat), die durch einen Abbindevorgang aushärten kann, und einem Matrixpolymer (u.a. Hyaluronsäure) und / oder einem komplexierenden Agens. JP 10 279421 A offenbart eine Zusammensetzung zur Verabreichung über die Haut, bestehend aus einem Derivat der L-Ascorbinsäure, einem Hyaluronsäure-Acetylester und ggf. einem medizinischen Agens.

Die Aufgabe der Erfindung ist es nun, eine pharmazeutische Zusammensetzung zur nasalen Anwendung von Fentanyl in Form einer Dispersion oder Lösung bereitzustellen, wobei die Zytotoxizität der pharmakologisch wirksamen Substanz herabgesetzt, bzw. aufgehoben wird.

Die Aufgabe wird erfindungsgemäß durch eine pharmazeutische Zusammensetzung zur nasalen Anwendung gelöst, welche Fentanyl als Wirkstoff mit einem Wirkstoffgehalt von 0.001 - 20.0 Gew.-%, jeweils bezogen auf das Gesamtgewicht der pharmazeutischen Zusammensetzung, Hyaluronsäure und/oder ihre Salze mit einem Molekulargewicht zwischen 2 Mio. und 3 Mio. Dalton und mit einem Gehalt an Hyaluronsäure und/oder ihren Salzen von 0.01 - 0.2 Gew.-% bezogen auf die Gesamtmenge der pharmazeutischen Zusammensetzung und gegebenenfalls mindestens einen Lösungsvermittler und/oder Hilfsstoff enthält.

Die erfindungsgemäße pharmazeutische Zusammensetzung kann mittels Vorrichtungen, die eine definierte Dosierung erzeugen können, auf die Nasenschleimhaut appliziert werden. Die bevorzugten Vorrichtungen umfassen gebräuchliche Pump- und Ventilsprays sowie Nasentropfen und Nasengele in geeigneten Behältnissen.
Die Zusammensetzung weist eine gute Resorption auf, da sie gut auf der Nasenschleimhaut haftet, zudem durch das Fliesverhalten der Hyaluronsäure eine gute Bioadhäsion erzielt wird und der Wirkstoff somit sehr leicht aus der Zusammensetzung von der Nasenschleimhaut resorbiert wird.

Das Problem des zytotoxischen Einflusses der pharmakologisch wirksamen Substanz in wässrigen Lösungen ergibt sich, völlig unerwartet und für den Fachmann nicht vorhersehbar, bei der erfindungsgemäßen Zusammensetzung nicht. Wie aus Abbildung 1 und Tabelle 1 klar ersichtlich, kommt es bei der Applikation einer Fentanyl umfassenden erfindungsgemäßen Zusammensetzung nur zu einem geringfügig verminderten Zellwachstum im Vergleich zur Kontrolle und der reinen Hyaluronsäurelösung, während bei Applikation einer Fentanyllösung die Zellzahl bedingt durch die Zytotoxizität eklatant vermindert wird.

**Tabelle 1: Deskriptive Statistik des Zellwachstums (Zellzahl / ml)**

| | N | Mean | Zellwachstum (%) | Minimum | Maximum | Std. Deviation | p* |
|---|---|---|---|---|---|---|---|
| Kontrolle | 19 | 424,85 | 100 | 366 | 499 | 39,72 | |
| Hyaluronsäure-Lösung | 19 | 424,48 | 99,91 | 381 | 474 | 29,88 | 0,931 |
| Fentenyl-Lösung | 19 | 37,07 | 8,73 | 5 | 75 | 17,70 | 0,001 |
| Fentanyl + Hyaluronsäure-Lösung | 19 | 398,28 | 93,75 | 314 | 503 | 52,58 | 0,080 |
| Fentanyl- Placebo | 19 | 185,07 | 43,56 | 53 | 284 | 66,93 | 0,001 |

| | | | | | | | |
|---|---|---|---|---|---|---|---|
| * U-Test vs. Kontrolle | | | | | | | |

Die Verträglichkeit der erfindungsgemäßen Zusammensetzung auf der Nasenschleimhaut ist sehr gut, und somit kann die Patientencompliance erhöht werden.

Ohne die Zilien zu zerstören, ihre Funktion wesentlich einzuschränken, oder eine Veränderung der chemischen Struktur des Wirkstoffes zu verursachen kann eine maximale Resorption erreicht werden.

Die Zusammensetzung ist bei Vorliegen einer Lösung gut filtrierbar, so dass durch eine Sterilfiltration (0,2 µm Porengröße) ohne großen Aufwand eine sterile Lösung hergestellt werden kann. Bei Vorliegen einer Dispersion kann die Sterilität ohne Probleme erreicht werden, indem zuerst die Hyaluronsäure-Lösung steril filtriert wird und anschließend die Dispersion im geschlossenen Kreislauf angesetzt wird. Die Herstellung ist einfach und kostengünstig, da keine weiteren Zusätze nötig sind.

Unter dem Begriff Hyaluronsäure werden Makromoleküle mit einer verschlungenen Spiralstruktur verstanden, die bereits in sehr niedrigen Konzentrationen polymere Gitter bilden und eine sehr hohe Wasserbindungskapazität haben.

Das Molekulargewicht der verwendeten Hyaluronsäuren liegt zwischen 2 und 3 Mio. Dalton, bevorzugt wird ein Molekulargewieht von 2,4 bis 2,9 Mio. Dalton.

Die erfindungsgemäße pharmazeutische Zusammensetzung kann eine Viskosität von 1-40 mPa·s aufweisen, bevorzugt eine Viskosität von 5-20 mPa·s, ganz besonders bevorzugt wird eine Viskosität von 8-15 mPa·s.

Die erfindungsgemäße pharmazeutische Zusammensetzung enthält Fentanyl, als Wirkstoffkomponente.

Die erfindungsgemäße pharmazeutische Zusammensetzung kann einen Wirkstoffgehalt an pharmazeutisch wirksamen Substanzen von jeweils, sei es einzeln und/oder in Kombination, 0.001-20.0 Gew%, bevorzugt 0.01-10.0 Gew.% aufweisen. Die Prozentangaben beziehen sich auf die Gesamtmenge der pharmazeutischen Zusammensetzung.

Die erfindungsgemäße pharmazeutische Zusammensetzung kann einen Gehalt an Hyaluronsäure und / oder ihren Derivaten von 0.01-0.2 Gew.% aufweisen. Die Prozentangaben beziehen sich auf die Gesamtmenge der pharmazeutischen Zusammensetzung.

Die erfindungsgemäße pharmazeutische Zusammensetzung kann gegebenenfalls noch Antioxidantien wie z.B. α-Tocopherol, α-Tocopherolester, Ascorbinsäure, Ascorbinsäureester (-myristat, -palmitat und -stearat), β-Carotin, Cystein, Acetylcystein, Folsäure (Vitamin-B₂-Gruppe), Phytinsäure, cis- und/ oder trans-Urocansäure, Karnosin (N-β-Alanin-L-Histidin), Histidin, Flavone, Flavonoide, Lycopin, Tyrosin, Gluthation, Gluthationester, α-Liponsäure, Ubichinon, Nordihydroguaiaretsäure (NDGA), Gallussäureester (Ethyl-, Propyl-, Octyl-, Dodecylgallat), Phosphorsäurederivate (Monophosphate, Polyphosphate), Butylhydroxytoluol (BHT), Butylhydroxyanisol (BHA), Tetraoxydimethylbiphenyl (TDBP), Polyalkohole, Citronensäure, Weinsäure, Edetinsäure (EDTA als Di-Na- oder Di-Na-Ca-Salz), Coniferylbenzoat und/oder deren Derivate enthalten, die die Aufnahme durch die Nasenschleimhaut fördern und/oder die Lösung zusätzlich stabilisieren.

Der Gehalt der gegebenenfalls zugefügten Antioxidantien kann 0,001-2 Gew.%, bezogen auf die Gesamtmenge der pharmazeutischen Zusammensetzung, betragen.

Die erfindungsgemäße pharmazeutische Zusammensetzung kann gegebenenfalls noch Lösungsvermittler wie z.B. Lysophosphatidylcholin, Lysophosphatidylglycerol, Phosphatidylethanolamin, Phosphatidylserin, Phosphatidylinosit(ol)e, Spingophospholipide, Natriumdodecylsulfat, Natriumcetylstearylsulfat, Natriumdioctylsulfosuccinat, Cetylstearylalkohol, Cetylalkohol, . Stearylalkohol, Cholesterol, Sorbitanmonooleat, Sorbitanmonopalmitat, Sorbitanmonostearat, Sorbitantrioleat, Sorbitantristearat, Sorbitanmonolaurat, Polysorbat 20, Polysorbat 60, Polysorbat 80, Polysorbat 40, Macrogol-1500-glyceroltriricinnoleat, Macrogol-Glycerolhydroxystearat, Macrogol-1000-Glycerolmonolaurat, Macrogol-1000.Glycerolmonolaurat, Macrogol-1000-Glycerolmonooleat, Macrogolstearat, Polyoxyl40stearat, Polyoxyl50stearat, Polyoxyl23laurylether, Polyoxyl20cetostearylether, Polyoxyl10olylether, Glycerolmonostearat und/ oder Poloxamer enthalten.

Diese Lösungsvermittler können eine Lösung des wasserlöslichen Wirkstoffes unterstützen bzw. ermöglichen und u.a. eine evtl. auftretende Adsorptionsverbindung des Wirkstoffes mit der Wandung des Behälters (Ablagerung) verhindern.

Die erfindungsgemäße pharmazeutische Zusammensetzung kann gegebenenfalls noch Resorptionsverstärker wie z.B. Dimethyl-β-Cyclodextrin, Permethyl-β-Cyclodextrin, Hydroxypropyl-β-Cyclodextrin, randomisiertes methyliertes β-Cyclodextrin, Carboxymethyl-β-Cyclodextrin, Maltosyl-β-Cycodextrin, γ-Cyclodextrin, Natriumtaurofusidat, Natriumglykocholat, Laureth-9 und/ oder α-Lecithin enthalten.

Die Erfindung wird durch das nachstehende Beispiel näher erläutert, ohne aber den Erfindungsumfang damit einzuschränken.

### Beispiel 1:

50 mg Fentanyl und 0,01 g Hyaluronsäure werden in 100 ml Wasser pharmazeutischer Qualität eingebracht. Diese Lösung wird über einen 0,2 µm Filter steril filtriert und in ein Pumpspray mit einem Dosisvolumen von 140 µl abgefüllt.

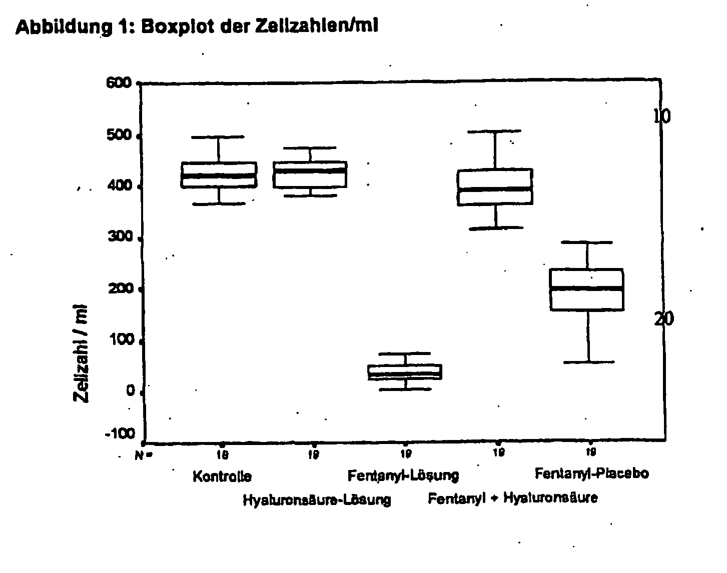

## Patentansprüche

1. Pharmazeutische Zusammensetzung zur nasalen Anwendung enthaltend Fentanyl als Wirkstoff mit einem Wirkstoffgehalt von 0.001 - 20.0 Gew.-%, jeweils bezogen auf das Gesamtgewicht der pharmazeutischen Zusammensetzung, Hyaluronsäure und/oder ihre Salze mit einem Molekulargewicht zwischen 2 Mio. und 3 Mio. Dalton und mit einem Gehalt an Hyaluronsäure und/oder ihren Salzen von 0.01 - 0.2 Gew.-% bezogen auf die Gesamtmenge der pharmazeutischen Zusammensetzung und gegebenenfalls mindestens einen Lösungsvermittler und/oder Hilfsstoff.

2. Pharmazeutische Zusammensetzung nach Anspruch 1, umfassend mindestens ein Antioxidationsmittel.

3. Pharmazeutische Zusammensetzung nach Anspruch 2, **gekennzeichnet durch** α-Tocopherol, α-Tocopherolester, Ascorbinsäure, Ascorbinsäureester, β-Carotin, Cystein, Acetylcystein, Folsäure, Phytinsäure, cis- und / oder trans-Urocansäure, Karnosin, Histidin, Flavone, Flavonoide, Lycopin, Tyrosin, Gluthation, Gluthationester, α-Liponsäure, Ubichinon, Nordihydroguaiaretsäure, Gallussäureester, Phosphorsäurederivate, Butylhydroxytoluol, Butylhydroxyanisol, Tetraoxydimethylbiphenyl, Polyalkohole, Zitronensäure, Weinsäure, Edetinsäure, EDTA als Di-Na- oder Di-Na-Ca-Salz und/oder Coniferylbenzoat als Antioxidationsmittel.

4. Pharmazeutische Zusammensetzung nach einem der vorangegangenen Ansprüche, umfassend mindestens einen Lösungsvermittler.

5. Pharmazeutische Zusammensetzung nach Anspruch 4, **gekennzeichnet durch** Lysophosphatidylcholin, Lysophosphatidylglycerol, Phosphatidylethanolamin, Phosphatidylserin, Phosphatidylinosit(ol)e, Spingophospholipide, Natriumdodecylsulfat, Natriumcetylstearylsulfat, Natriumdioctylsulfosuccinat, Cetylstearylalkohol, Cetylalkohol, Stearylalkohol, Cholesterol, Sorbitanmonooleat, Sorbitanmonopalmitat, Sorbitanmonostearat, Sorbitantrioleat, Sorbitantristearat, Sorbitanmonolaurat, Polysorbat 20, Polysorbat 60, Polysorbat 80, Polysorbat 40, Macrogol-1500-glyceroltriricinnoleat, Macrogol-Glycerolhydroxystearat, Macrogol-1000-Glycerolmonolaurat, Macrogol-1000-Glycerolmonooleat, Macrogolstearat, Polyoxy-140-stearat, Polyoxy-150-stearat, Polyoxy-123-laurylether, Polyoxy-120-cetostearylether, Polyoxy-110-olylether, Glycerolmonostearat und/oder Poloxamer als Lösungsvermittler.

6. Pharmazeutische Zusammensetzung nach einem der vorangegangenen Ansprüche, umfassend mindestens einen Resorptionsverstärker.

7. Pharmazeutische Zusammensetzung nach Anspruch 6, **gekennzeichnet durch** Dimethyl-β-Cyclodextrin, Permethyl-β-Cyclodextrin, Hydroxypropyl-β-Cyclodextrin, randomisiertes methyliertes β-Cyclodextrin, Carboxymethyl-β-Cyclodextrin, Maltosyl-β-Cyclodextrin, γ-Cyclodextrin, α-Cyclodextrin, Natriumtaurofusidat, Natriumglykocholat, Laureth-9 und/oder α-Lecithin als Resorptionsverstärker.

8. Pharmazeutische Zusammensetzung nach einem der vorangegangenen Ansprüche, **dadurch gekennzeichnet, dass** die pharmazeutische Zusammensetzung mittels geeigneter Applikationssysteme, bevorzugt als Spray, in die Nase eingebracht wird.

## Claims

1. Pharmaceutical composition for nasal application containing fentanyl as active ingredient having a content of active ingredient of 0.001 - 20.0 wt.%, in relation to the total weight of the pharmaceutical composition, hyaluronic acid and/or its salts with a molecular weight of between 2 million and 3 million daltons and having a content of hyaluronic acid and/or its salts of 0.01 - 0.2 wt.%, in relation to the total amount of the pharmaceutical composition, and optionally at least one solubilizer and/or adjuvant.

2. Pharmaceutical composition according to claim 1 comprising at least one antioxidant.

3. Pharmaceutical composition according to claim 2 **characterized by** α-tocopherol, α-tocopherol ester, ascorbic acid, ascorbic acid ester, β-carotene, cysteine, acetylcysteine, folic acid, phytic acid, cis- and/or trans-urocanic acid, carnosine, histidine, flavones, flavonoides, lycopene, tyrosine, glutathion, glutathion ester, α-lipoic acid, ubiquinone, nordihydroguaiaretic acid, gallic acid ester, phosphoric acid derivatives, butylhydroxytoluene, butylated hydroxyanisole, tetraoxydimethylbiphenyl, polyalkohols, citric acid, tartaric acid, ethylenediaminetetraacetic acid, EDTA as di-Na- or di-Na-Ca-salt and/or coniferylbenzoate as antioxidant.

4. Pharmaceutical composition according to one of the previous claims comprising at least one solubilizer.

5. Pharmaceutical composition according to claim 4 **characterized by** lysophosphatidylcholine, lysophosphatidylglycerol, phosphatidylethanolamine, phosphatidylserine, phosphatidylinosit(ol)e, spingophospholipides, sodium dodecyl sulfate, sodium cetylstearyl sulphate, sodium dioctylsulphosuccinate, cetylstearyl alcohol, cetyl alcohol, stearyl alcohol, cholesterol, sorbitan monooleate, sorbitan monopalmitate, sorbitan monostearate, sorbitan trioleate, sorbitan tristearate, sorbitan monolaurate, polysorbate 20, polysorbate 60, polysorbate 80, polysorbate 40, macrogol-1500-glyceroltriricinoleate, macrogol-glycerolhydroxystearate, macrogol-1000-glycerolmonolaurate, macrogol-1000-glycerolmonooleate, macrogolstearate, polyoxy-140-stearate, polyoxy-150-stearate, polyoxy-123-laurylether, polyoxy-120-cetostearylether, polyoxy-110-olylether, glycerolmonostearate, and/or poloxamer as solubilizer.

6. Pharmaceutical composition according to one of the previous claims comprising at least one resorption enhancer.

7. Pharmaceutical composition according to claim 6 **characterized by** dimethyl-β-cyclodextrin, permethyl-β-cyclodextrin, hydroxypropyl-β-cyclodextrin, randomized methylated β-cyclodextrin, carboxymethyl-β-cyclodextrin, maltosyl-β-cyclodextrin, γ-cyclodextrin, α-cyclodextrin, sodium taurofusidate, sodium glycocholate, laureth-9 and/or α-lecithin as resorption enhancer.

8. Pharmaceutical composition according to one of the previous claims **characterized by** that the pharmaceutical composition is introduced into the nose by use of suitable application systems, preferably as spray.

## Revendications

1. Composition pharmaceutique pour application nasale, contenant du fentanyl en tant que principe actif, selon une teneur en principe actif comprise entre 0,001 et 20,0 % en masse, respectivement sur la base de la masse totale de la composition pharmaceutique, de l'acide hyaluronique et/ou ses sels présentant une masse moléculaire comprise entre 2 millions et 3 millions de daltons, selon une teneur en acide hyaluronique et/ou en ses sels comprise entre 0,01 et 0,2 en masse, sur la base d e la masse totale de la composition pharmaceutique, et éventuellement un intermédiaire de solubilisation et/ou un adjuvant.

2. Composition pharmaceutique selon la revendication 1, comprenant au moins un agent antioxydant.

3. Composition pharmaceutique selon la revendication 2, **caractérisée par** de l'α-tocophérol, un ester d'α-tocophérol, de l'acide ascorbique, un ester d'acide ascorbique, du β-carotène, de la cystéine, de l'acétylcystéine, de l'acide folique, de l'acide phytique, de l'acide cis- et/ou trans-urocanique, de la carnosine, de l'histidine, des flavones, des flavonoïdes, de la lycopine, de la tyrosine, du gluthation, un ester de gluthation, de l'acide α-liponique, de l'ubiquinone, de l'acide nordihydroguaïarétique, un ester d'acide gallique, des dérivés d'acide phosphorique, du butylhydroxytoluène, du butylhydroxyanisole, du tétraoxydiméthylbiphényle, des polyalcools, de l'acide citrique, de l'acide tartrique, de l'acide édétinique, de l'EDTA en tant que sel de di-Na ou de di-Na-Ca et/ou du benzoate de coniféryle en tant qu'antioxydant.

4. Composition pharmaceutique selon l'une des revendications précédentes, comprenant au moins un intermédiaire de solubilisation.

5. Composition pharmaceutique selon la revendication 4, **caractérisé par** de la lysophosphatidylcholine, du lysophosphatidylglycérol, de la phosphatidyléthanolamine, de la phosphatidylsérine, des phosphatidylinositols, des spingophospholipides, du dodécylsulfate de sodium, du cétylstéarylsulfate de sodium, du dioctylsulfosuccinate de sodium, un alcool de cétylstéaryle, un alcool de cétyle, un alcool de stéaryle, du cholestérol, du monooléate de sorbitane, du monopalmitate de sorbitane, du monostéarate de sorbitane, du trioléate de sorbitane, du tristéarate de sorbitane, du monolaurate de sorbitane, du polysorbate 20, du polysorbate 60, du polysorbate 80, du polysorbate 40, du glycéroltriricinoléate de macrogol 1500, du glycérolhydroxystéarate de macrogol, du glycérolmonolaurate de macrogol 1000, du glycérolmonooléate de macrogol 1000, du stéarate de macrogol, du stéarate de polyoxy 140, du stéarate de polyoxy 150, du lauryléther de polyoxy 123, du cétostéaryléther de polyoxy 120, de l'olyléther de polyoxy 110, du monostéarate de glycérol et/ou un poloxamère en tant qu'intermédiaire de solubilisation.

6. Composition pharmaceutique selon l'une des revendications précédentes, comprenant au moins un renforçateur de résorption.

7. Composition pharmaceutique selon la revendication 6, **caractérisée par** de la diméthyl-β-cyclodextrine, de la perméthyl-β-cyclodextrine, de l'hydroxypropyl-β-cyclodextrine, de I a β-cyclodextrine méthylée aléatoire, de la carboxyméthyl-β-cyclodextrine, de la maltosyl-β-cyclodextrine, de la γ-cyclodextrine, de l'α-cyclodextrine, du taurofusidate de sodium, du glycocholate de sodium, du laureth 9 et/ou de l'α-lécithine en tant que renforçateur de résorption.

8. Composition pharmaceutique selon l'une des revendications précédentes, **caractérisée en ce que** la composition pharmaceutique est appliquée dans le nez au moyen de systèmes d'application appropriés, de préférence sous la forme d'un spray.
